# EUROPEAN PATENT SPECIFICATION

(11) **EP 2 035 076 B1**
(45) Date of publication and mention of the grant of the patent: **08.09.2010**
(21) Application number: 07722674.4
(22) Date of filing: 20.06.2007
(51) Int. Cl.: A61M 39/10, A61M 39/00

(54) **DEVICE CONNECTOR AND A SYSTEM COMPRISING A MEDICAL DEVICE CONNECTOR**
VERBINDER FÜR VORRICHTUNGEN UND SYSTEM MIT EINEM VERBINDER FÜR MEDIZINISCHE VORRICHTUNGEN
CONNECTEUR DE DISPOSITIF ET SYSTÈME COMPRENANT UN CONNECTEUR DE DISPOSITIF MÉDICAL

(30) Priority: 20.06.2006 DK 200600837; 20.06.2006 US 814905 P
(43) Date of publication of application: 18.03.2009
(73) Proprietor: Coloplast A/S, 3050 Humlebæk (DK)
(72) Inventor: KAERN, Viggo Aaberg, 2000 Frederiksberg (DK)
(86) International application number: PCT/DK2007/000297
(87) International publication number: WO 2007/147410

(56) References cited:
- US-A- 4 676 530
- US-A- 4 969 879
- US-A1- 2005 245 899
- US-A1- 2006 004 345
- US-A1- 2006 025 751

## Description

The present invention generally relates to a medical device connector for transmission of fluids, in particular bodily fluids, e.g. blood and urine, which may be used in systems for draining or introducing fluids.

People using catheters may during daytime have a urine bag connected to a catheter. This urine bag only has a limited size due to discretion as it e.g. may be fixed to a leg of the user. The urine bag may then be emptied when needed, though during nighttimes the capacity of the urine bag may not be large enough. In order to solve this problem another collection bag can be coupled to the urine bag e.g. via a piece of flexible tube allowing an outlet of the urine bag and an inlet in the collection bag to be sealingly secured. In this way the capacity is increased. However, in such a solution it may be difficult to connect the tube and the insert since the flexibility of the tube piece is limited, as it should still provide that the tube is sealingly secured with the outlet respectively the insert. Also, this solution adds extra costs to the draining system due to the tube piece. Furthermore, the tube piece may kink which, if it is not noticed that urine is not drained from the urine bag and thus a pressure is build up, in the worst case may damage the kidneys.

Thus, it is an object of the invention to provide a medical device connector that prevents kinking.

It is a further object to provide a medical device connector that is easy to connect with an insert.

These and other objects are fulfilled by the invention.

In a first aspect of the invention a medical device connector for transmission of fluids in fluidic systems is provided, the connector comprising
a tubular engagement section having
a stabilisation part, and
a securing part comprising a soft flexible first material, which when in use sealingly secures engagement with an insert,
wherein the combination of the stabilisation part and the securing part provides that said engagement section is more stabile in a longitudinal direction than in a radial direction, and that said engagement section is more flexible in the radial direction than in the longitudinal direction. It is an advantage that the connector allows for flexibility in the radial direction while at the same time being more stabile in the longitudinal direction that is in the connection direction, as this provides for flexibility when connecting the medical device connector with an insert and at the same time it prevents kinking. Also, this provides that the engagement section exerts a clamp force on the insert.

By tubular is meant that the engagement section has a hollow elongated structure that provides a fluid to pass. The insert may e.g. be an inlet or an outlet.

In an embodiment of the invention a connector is provided wherein the tubular engagement section is funnel shaped or frusto-conical or another diverging shape facilitating the connection of the insert with the connector. Alternatively, the tubular engagement section could be stepped that is with different cross section areas.

In another embodiment of the invention a connector is provided, wherein the tubular engagement section is essentially circular. It is an advantage since there is no orientation requirement to the connector when connecting the engagement section with an insert. The tubular engagement section could also have any other shapes like oval.

In yet another embodiment a connector is provided, wherein the stabilisation part comprises a stabilisation geometry. When the connector is in engagement with an insert, at least part of the stabilisation geometry overlaps part of the insert for in this way to prevent kinking. The stabilisation geometry may e.g. comprise a number of beams, such as between 2 and 12 beams, e.g. between 3 and 6 beams, preferably 4 beams. It is advantageous to use a stabilisation geometry comprising beams since this provides more flexibility in the distal end of the beam than in the proximal end of the beam, such that the connector will be easy to connect while at the same time the beams will avoid kinking. In an embodiment at least one beam has a proximal end and a distal end so that the width of the beam at the proximal end is wider than the width at the distal end. The distal end of the beam may e.g. be rounded. In this way the risk that the beams rupture the securing part is reduced. In another embodiment at least one beam has a thinner material thickness at the distal end than at the proximal end. This also reduces the risk of rupturing the securing part.

In another embodiment of the invention a connector is provided, wherein the stabilisation part is made of a second material. The securing section may be made of a thermoplastic elastomer, and the stabilisation part is made of a thermoplastic material. In this way, the stabilisation part is made of a more stiff material than the sealing section facilitating the wanted flexibility in the radial direction and at the same time the stiffness in the longitudinal direction, i.e. the connection direction. As an example, the securing section is made of a styrenic based thermoplastic elastomer, e.g. Kraton®, and the stabilisation part is made of polypropylene, since this provides the wanted mechanical properties, flexibility in the radial direction and at the same time the stiffness in the longitudinal direction, while reducing the environmental impact.

In a preferred embodiment of the invention, the connector comprises engagement means manufactured by multi-component moulding.

In another embodiment of the invention, a connector wherein the stabilisation part is at least partly integrated in the securing part is provided. This provides for easy cleaning of the connector that is important in medical use.

Also in another embodiment of the invention, the connector may comprise a valve and a catch means.

In a second aspect of the invention, a medical bag for fluids is provided, in particular a urine bag, wherein the bag comprises a medical device connector according to any of the embodiments of the medical device connector according to the first aspect of the invention. This is an advantage since e.g. the piece of tube connecting two urine bags (as described earlier) will be dispensable.

In a third aspect of the invention, a medical collection system for fluids is provided, the system comprising a connector according to the first aspect of the invention and an insert, wherein connection between two devices for handling fluids is established by the medical device connector. A device for handling fluids may e.g. be used for containing or transmitting fluids e.g. a catheter, a collection bag for bodily fluids such as urine, or a tube.

The invention is disclosed more in detail with reference to the drawings in which
Fig. 1 shows a medical device connector according to an embodiment of the invention;
Fig. 2a, Fig. 2b and Fig. 2c show engagement sections of a medical device connector;
Fig. 3a, Fig. 3b, Fig. 3c and Fig. 3d show various embodiments of the cross sections of the engagement section according to the invention; and
Fig. 4a and Fig. 4b illustrate connection of an insert to a medical device connector according to the invention.

The invention is now explained more in detail with reference to the drawings showing preferred embodiments of the invention.

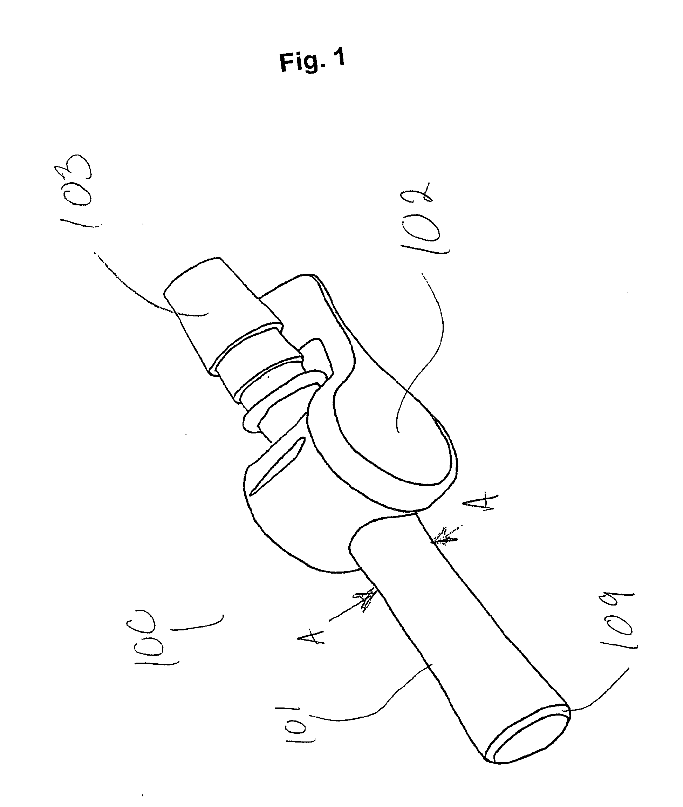

Fig. 1 shows an embodiment of a medical device connector 100 according to the invention. The connector 100 comprises an engagement section 101 that is adapted to receive an insert. The engagement section 101 is hollow, preferably tubular, having a wall thickness of about 1 mm and with a tapered end 109. The engagement section may be ended in other ways, e.g. having a rounded end. In the embodiment shown in the figure the medical device connecter 100 comprises a catch member 103 and a valve system 102. However, the medical device connector 100 could be made without the valve system. Also the medical device connector 100 could be made with another engagement section, e.g. like the engagement section 101, instead of the catch element 103.

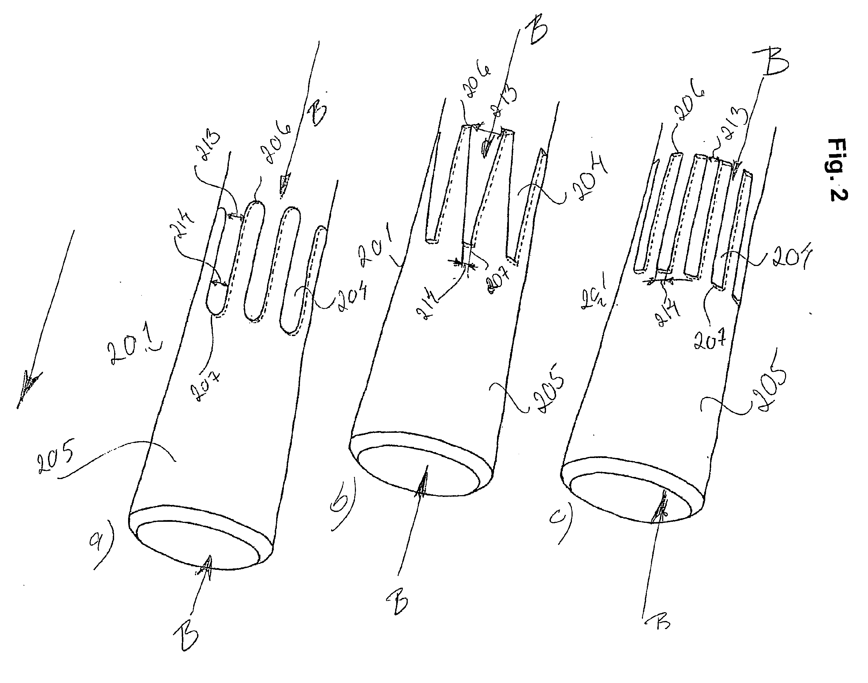

In Fig. 2a, 2b and 2c various embodiments of an engagement section 201 are illustrated. All of the embodiments comprise a securing part 205 made of a flexible material and a stabilisation geometry in this case a number of beams 204 that provide for stiffening the engagement means in the longitudinal direction while still allowing flexibility in the radial direction. The arrow A in the figure indicates the longitudinal direction. The beams 204 further provide for flexibility in the radial direction of the engagement section 201. The number of beams may be any number as long as they provide for the mechanical properties as described above, e.g. may the number of beams be between 2 and 12 such as 4. Fig. 2 shows various embodiments of the geometrical shape of the beams. In Fig. 2 the lengths of the beams are the same but in another embodiment the lengths of the beams may vary. The beams may e.g. have a length between 5mm and 30mm, and a width between 1 mm 5mm. The length of the securing part is typically between 10mm - 70mm. The lengths of the beams may e.g. be between ¼ of the length of the securing part to the complete length of the securing part. In all of the figures a cross section B-B is indicated with arrows.

In Fig. 2a the beam has a curved distal end 207. In this way the risk is reduced that the beam ruptures the securing part 205 due to wear. Also the connections 206 of the beams 204 in Fig. 2a are curved. The beams 204 in Fig. 2c have straight ends 214 and straight connections 206. The beams 204 in Fig. 2b have a varying width so that the width at the distal end of the beam 214 is smaller than that of the proximal end 213 of the beam 204, which makes it easier to take the cast of after moulding. Even though the beams 204 in the embodiment in Fig. 2b have straight distal ends 214 and connections 206, they can have other shapes such as rounded. The width of the connections 206 may be larger than the width of the proximal end 213 and vice versa.

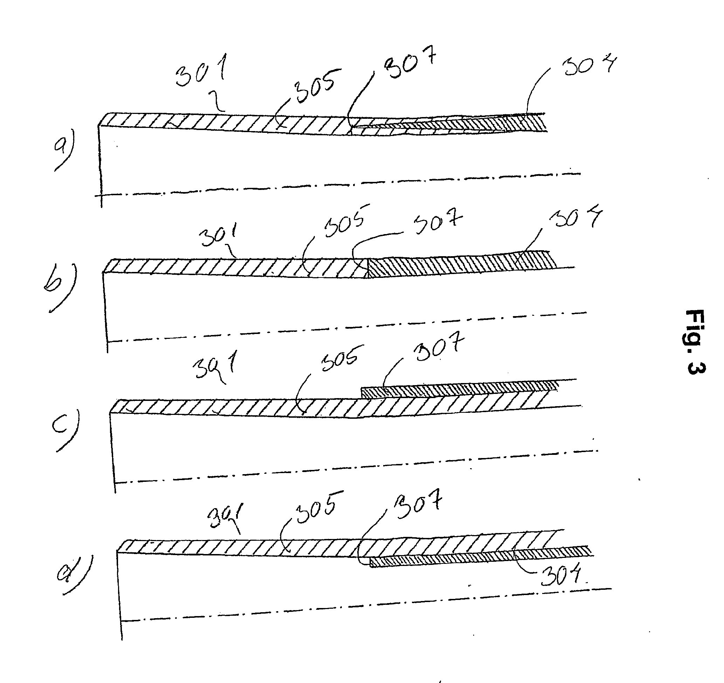

Fig. 3a, 3b, 3c and 3d all show cross sections of the engagement section 301 (like the ones B-B shown in Fig.2). The figure shows various examples of combinations of the securing part and the beams 304 of the stabilisation part.

In Fig. 3a and Fig. 3b the beams are integrated in the securing part so that the engagement section appears to be in one piece, i.e. there is no tactile transition between the securing part and the stabilisation part. Fig. 3a shows an embodiment where the thickness of the beam 304 varies from having the same thickness as the wall thickness of the securing part to a pointed distal end 307 that may be rounded. In Fig. 3b the beam has the same material thickness as the wall thickness of the securing part.

Fig. 3c and Fig. 3d show embodiments where the beams 304 are on top or underneath the securing section 305. In these embodiments there is a tactile transition between the beams 304 and the securing part. Both figures show an abrupt transition between the beams and the securing part. In other embodiments, the transition between the beams and the securing part could be smooth. This is an advantage since it provides for an easier cleaning of the engagement section.

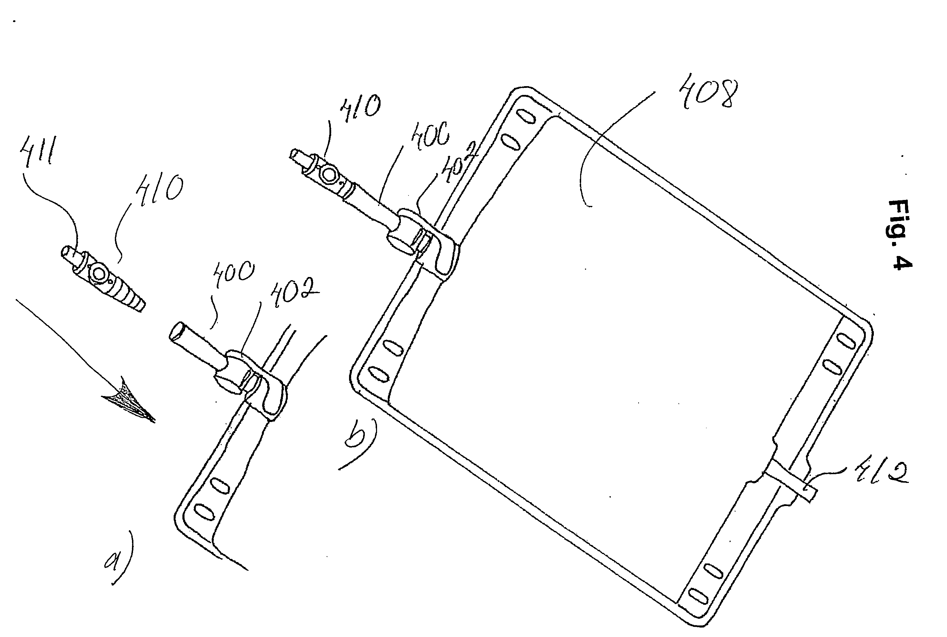

Fig. 4 illustrates arrangement of an embodiment of the invention where the medical device connector 400 comprises a valve system 402. In this arrangement the medical device connector 400 forms an outlet of a collection bag for urine 408 having a tube member, which allows fluid to pass into the collection bag. Fig. 4a shows an insert 410, in this case an inlet connected to a tube 411. The insert 410 is entered into the engagement section 400 in the longitudinal direction, which in the connection direction is indicated by the arrow in the figure. Fig. 4b shows how the insert 410 is secured with the engagement section 400.

In all of the embodiments the securing section 205, 305 could be made of a thermoplastic material such as a thermoplastic elastomer, e.g. a triblock copolymer or a compound with different additives. Preferably a styrenic based thermoplastic elastomer like Kraton® is used. The stabilisation part could be made of a more stiff plastic than the securing part, such as a thermoplastic material like polypropylene. However, in all of these embodiments it is important that the securing section 205, 305 is made of an elastic material that provides flexibility so that the engagement section can be sealingly engaged with an insert. Also, a Poly Vinyl Chloride (PVC) with a softener may be used for the stabilisation part and the securing part. The mechanical properties can be obtained by the amount of the softener.

In the embodiments where the beams 204, 304 in the stabilisation part are integrated in the securing part 205, 305 the thermoplastic material for the stabilisation part should be stiffer with a higher modulus of elasticity (Young's modulus) than the one for the securing part. Another way to obtain a higher stiffness or elasticity is by varying the wall thickness in areas, e.g. as illustrated in the embodiments shown in Fig. 3c and Fig. 3d. The beams 304 in Fig. 3c and Fig. 3d provide a higher wall thickness when combined with the securing part, which then result in a higher stiffness than the stiffness of the stabilization part. In this way the stabilisation part could be made of the same material as the securing part or even of a material with a lower modulus of elasticity as long as flexibility is provided in the radial direction of the engagement section together with the stiffness in the longitudinal direction.

An advantage of using the same material of the securing part and the stabilisation section is that the costs of the investment in the injection-moulding tools would be lower and further a common injection-moulding unit can be used. Another way of achieving a higher stiffness in predetermined areas could be by overmoulding. This would be an advantage since the investments in two injection-moulding tools with common injection-moulding units would be lower than the investments in one complex injection-moulding tool.

Yet another way of manufacturing could be by multi-component injection moulding. An advantage about this method is that the connector will be manufactured in one step and that the cycle time will be low.

The manufacturing of the connector may also be a combination of injection moulding and a cutting process like milling and turning, so that first the stabilisation part will be moulded and the stabilisation geometry will be cut, and hereafter the securing part can be moulded together with the stabilisation part thereby forming the engagement section. It is an advantage to use such a process producing small quantities of the connector.

For all of the embodiments the securing part is adapted to adjust and secure to the insert in order to provide sealing, thereby preventing leakage and that the medical device connector is fixed with the insert due to the friction between the insert and the securing part.

## Claims

1. Medical device connector for transmission of fluids in fluidic systems, the connector comprising
a tubular engagement section having
a stabilisation part, and
a securing part comprising a soft flexible first material, which when in use sealingly secures engagement with an insert,
wherein the combination of the stabilisation part and the securing part provides that said engagement section is more stabile in a longitudinal direction than in a radial direction and that said engagement section is more flexible in the radial direction than in the longitudinal direction.

2. A connector according to any of the preceding claims, wherein the tubular engagement section is funnel shaped.

3. A connector according to any of the preceding claims, wherein the tubular engagement section is essentially circular.

4. A connector according to any of the preceding claims, wherein the stabilisation part comprises a stabilisation geometry.

5. A connector according to claim 4, wherein the stabilisation geometry comprises a number of beams.

6. A connector according to any of the claims 4 or 5, wherein the stabilisation part comprises between 2 and 12 beams.

7. A connector according to any of the claims 4-6, wherein at least one beam has a proximal end and a distal end so that the width of the beam at the proximal end is wider than the width at the distal end.

8. A connector according to claim 7, wherein the distal end of the beam is rounded.

9. A connector according to any of the claims 4-8, wherein at least one beam has a thinner material thickness at the distal end than at the proximal end.

10. A connector according to any of the preceding claims, where the stabilisation part is made of a second material.

11. A connector according to any of the preceding claims, wherein the securing section is made of a thermoplastic elastomer and the stabilisation part is made of a thermoplastic material.

12. A connector according to any of the preceding claims, wherein the securing section is made of a styrenic based thermoplastic elastomer and the stabilisation part is made of polypropylene.

13. A connector according to any of the preceding claims, wherein the engagement means is manufactured by multi-component moulding.

14. A connector according to any of the preceding claims, wherein the stabilisation part is at least partly integrated in the securing part.

15. A connector according to any of the preceding claims, further comprising a valve and a catch means.

16. A medical bag for fluids, in particular a urine bag, wherein the bag comprises a medical device connector according to any of the claims 1-15.

17. A medical collection system for fluids, the system comprising a medical device connector according to any of the claims 1-15 and an insert, wherein connection between two devices for handling fluids is established by the medical device connector.

## Patentansprüche

1. Verbinder für medizinische Vorrichtungen zum Durchleiten von Fluids in Fluidsystemen, wobei der Verbinder aufweist:
einen rohrförmigen Aufnahmeabschnitt, der aufweist:
einen Stabilisierungsteil und
einen Befestigungsteil, der ein weiches, flexibles erstes Material aufweist, das bei der Verwendung einen dichten Eingriff mit
einem Einsetzteil sicherstellt,
wobei die Kombination des Stabilisierungsteils mit dem Befestigungsteil dazu führt, dass der Aufnahmeabschnitt in einer Längsrichtung stabiler ist als in einer Radialrichtung und der Aufnahmeabschnitt in der Radialrichtung flexibler ist als in der Längsrichtung.

2. Verbinder nach einem der vorhergehenden Ansprüche, bei dem der rohrförmige Aufnahmeabschnitt trichterförmig ist.

3. Verbinder nach einem oder mehreren der vorhergehenden Ansprüche, bei dem der rohrförmige Aufnahmeabschnitt im Wesentlichen kreisförmig ist.

4. Verbinder nach einem oder mehreren der vorhergehenden Ansprüche, bei dem der Stabilisierungsteil eine stabilisierende Geometrie aufweist.

5. Verbinder nach Anspruch 4, bei dem die stabilisierende Geometrie eine Anzahl von Verstärkungsstreben umfasst.

6. Verbinder nach Anspruch 4 oder 5, bei dem der Stabilisierungsteil 2 bis 12 Verstärkungsstreben aufweist.

7. Verbinder nach einem oder mehreren der Ansprüche 4 bis 6, bei dem mindestens eine Verstärkungsstrebe ein proximales Ende und ein distales Ende derart aufweist, dass die Breite der Verstärkungsstrebe am proximalen Ende größer ist als die Breite am distalen Ende.

8. Verbinder nach Anspruch 7, bei dem das distale Ende der Verstärkungsstrebe abgerundet ist.

9. Verbinder nach einem oder mehreren der Ansprüche 4 bis 8, bei dem mindestens eine Verstärkungsstrebe am distalen Ende eine geringere Materialstärke aufweist als am proximalen Ende.

10. Verbinder nach einem oder mehreren der vorhergehenden Ansprüche, bei dem der Stabilisierungsteil aus einem zweiten Material besteht.

11. Verbinder nach einem oder mehreren der vorhergehenden Ansprüche, bei dem der Befestigungsabschnitt aus einem thermoplastischen Elastomer und der Stabilisierungsteil aus einem thermoplastischen Material hergestellt sind.

12. Verbinder nach einem oder mehreren der vorhergehenden Ansprüche, bei dem der Befestigungsabschnitt aus einem thermoplastischen Elastomer auf Styrolbasis und der Stabilisierungsteil aus Polypropylen hergestellt sind.

13. Verbinder nach einem oder mehreren der vorhergehenden Ansprüche, bei dem die Aufnahmemittel durch Mehrkomponenten-Spritzgießen hergestellt sind.

14. Verbinder nach einem oder mehreren der vorhergehenden Ansprüche, bei dem der Stabilisierungsteil zumindest teilweise im Befestigungsteil integriert ist.

15. Verbinder nach einem oder mehreren der vorhergehenden Ansprüche, der ferner ein Ventil und eine Befestigungseinrichtung aufweist.

16. Medizinischer Beutel für Fluids, insbesondere ein Urinbeutel, wobei der Beutel einen Verbinder für medizinische Vorrichtungen nach einem oder mehreren der Ansprüche 1 bis 15 aufweist.

17. Medizinisches Sammelsystem für Fluids, das einen Verbinder für medizinische Vorrichtungen nach einem oder mehreren der Ansprüche 1 bis 15 sowie ein Einsetzteil aufweist, wobei die Verbindung zwischen zwei Vorrichtungen zur Handhabung von Fluids durch den Verbinder für medizinische Vorrichtungen gebildet wird.

## Revendications

1. Connecteur de dispositif médical pour la transmission de fluides dans des systèmes fluidiques, le connecteur comprenant une section d'enclenchement tubulaire possédant
une partie de stabilisation, et
une partie de fixation comprenant un premier matériau mou flexible, qui, lors de l'utilisation, fixe la section d'enclenchement de manière étanche sur un embout,
dans lequel la combinaison de la partie de stabilisation et de la partie de fixation prévoit que ladite section d'enclenchement présente ne stabilité plus importante dans une direction longitudinale que dans une direction radiale et que ladite section d'enclenchement présente une flexibilité plus importante dans la direction radiale que dans la direction longitudinale.

2. Connecteur selon l'une quelconque des revendications précédentes, dans lequel la section d'enclenchement tubulaire a une forme d'entonnoir.

3. Connecteur selon l'une quelconque des revendications précédentes, dans lequel la section d'enclenchement tubulaire est essentiellement circulaire.

4. Connecteur selon l'une quelconque des revendications précédentes, dans lequel la partie de stabilisation présente une géométrie de stabilisation.

5. Connecteur selon la revendication 4, dans lequel la géométrie de stabilisation comporte un certain nombre de bandes.

6. Connecteur selon l'une quelconque des revendications 4 ou 5, dans lequel la partie de stabilisation comprend entre 2 et 12 bandes.

7. Connecteur selon l'une quelconque des revendications 4-6, dans lequel au moins une bande présente une extrémité proximale et une extrémité distale de telle sorte que la largeur de la bande à l'extrémité proximale est plus large que la largeur à l'extrémité distale.

8. Connecteur selon la revendication 7, dans lequel l'extrémité distale de la bande est arrondie.

9. Connecteur selon l'une quelconque des revendications 4-8, dans lequel au moins une bande présente une épaisseur de matériau plus fine à l'extrémité distale qu'à l'extrémité proximale.

10. Connecteur selon l'une quelconque des revendications précédentes, où la partie de stabilisation est composée d'un second matériau.

11. Connecteur selon l'une quelconque des revendications précédentes, dans lequel la partie de fixation est composée d'un élastomère thermoplastique et la partie de stabilisation est composée d'un matériau thermoplastique.

12. Connecteur selon l'une quelconque des revendications précédentes, dans lequel la partie de fixation est composée d'un élastomère thermoplastique à base de styrène et la partie de stabilisation est composée de polypropylène.

13. Connecteur selon l'une quelconque des revendications précédentes, dans lequel le moyen d'enclenchement est fabriqué grâce au moulage de plusieurs composants.

14. Connecteur selon l'une quelconque des revendications précédentes, dans lequel la partie de stabilisation est au moins partiellement intégrée dans la partie de fixation.

15. Connecteur selon l'une quelconque des revendications précédentes, comprenant en outre une soupape et un moyen de retenue.

16. Poche médicale pour fluides, notamment une poche urinaire, la poche comprenant un connecteur de dispositif médical selon l'une quelconque des revendications 1-15.

17. Système de collecte de fluides à usage médical, le système comprenant un connecteur de dispositif médical selon l'une quelconque des revendications 1-15 et un embout, la connexion entre deux dispositifs de manipulation de fluides étant établie par le connecteur de dispositif médical.
